# EUROPEAN PATENT SPECIFICATION

(11) **EP 2 762 186 B1**
(45) Date of publication and mention of the grant of the patent: **18.04.2018**
(21) Application number: 14166731.1
(22) Date of filing: 25.06.2010
(51) Int. Cl.: A61M 11/02

(54) **Aerosol therapy device**
Vorrichtung für Aerosoltherapie
Dispositif de thérapie par aérosol

(43) Date of publication of application: 06.08.2014
(62) Divisional of application: 10747499.1
(73) Proprietor: MED 2000 S.r.l., 25015 Desenzano del Garda (BS) (IT); Fraccaroli, Davide, 25015 Desenzano del Garda, Brescia (IT)
(72) Inventor: FRACCAROLI, Davide, 25015 Desenzano del Garda, Brescia (IT)
(74) Representative: Chimini, Francesco

(56) References cited:
- WO-A1-2007/122578

## Description

The present invention relates to an aerosol-therapy device of the type comprising a casing, a compressor group mounted in said casing and air intake means and air delivery means respectively to and from said compressor group. The compressor group comprises an electric motor and a compressor device which can be activated by said motor to draw in air from the outside environment and deliver it to a dispenser device, respectively through said air intake and delivery means.

Devices having such characteristics are known for example in document US6318360. Document WO 2007/122578 discloses a compressor/aspirator having features as in the preamble of the appended claim 1.

The purpose of the present invention is to propose an aerosol-therapy device in which the assembly of the compressor group and its fitting into the casing of the device result considerably simplified, with a consequent saving of time and of assembly costs of the whole device.

Another purpose of the invention is to make available an aerosol-therapy device wherein the compressor group is composed of a smaller number of components than the known groups, is more reliable and does not require maintenance.

Such purposes are achieved by an aerosol-therapy device according to claim 1. The dependent claims describe preferred or advantageous embodiments of the device.

The characteristics and advantages of the device according to the invention will however be evident from the description below, made by way of a nonlimiting example with reference to the attached figures, wherein:

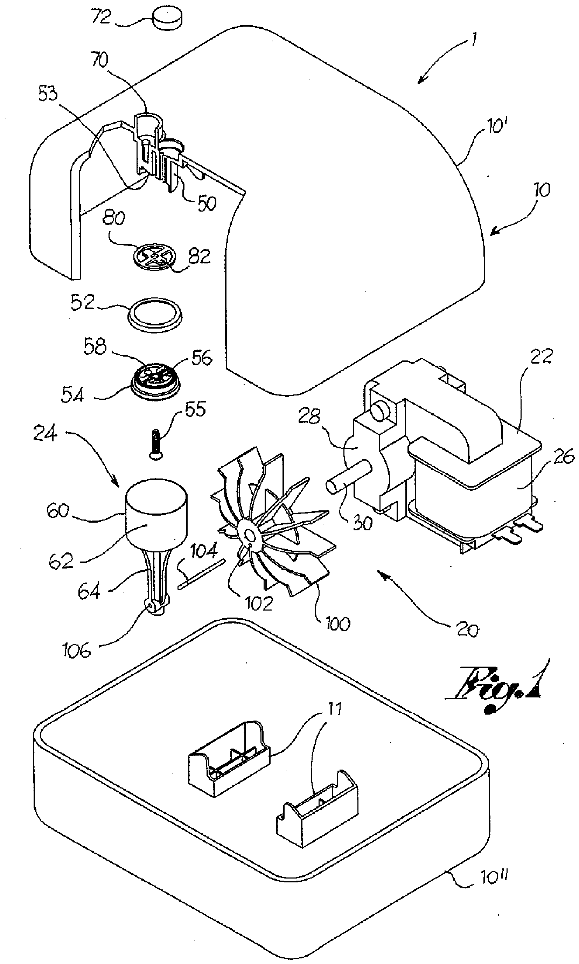

- figure 1 is an exploded perspective view of the aerosol-therapy device according to the invention;

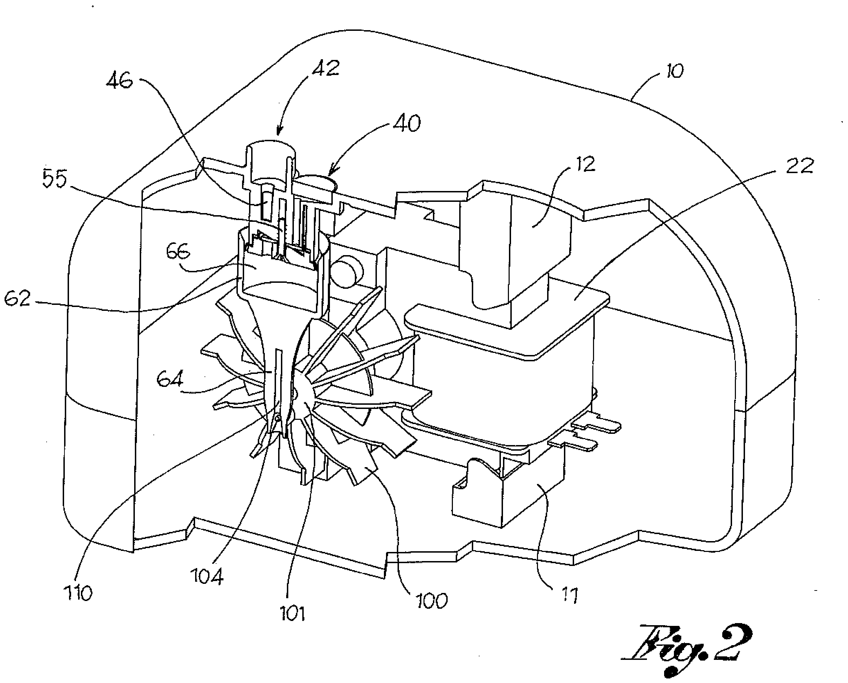

- figure 2 is a through-section of the assembled device;

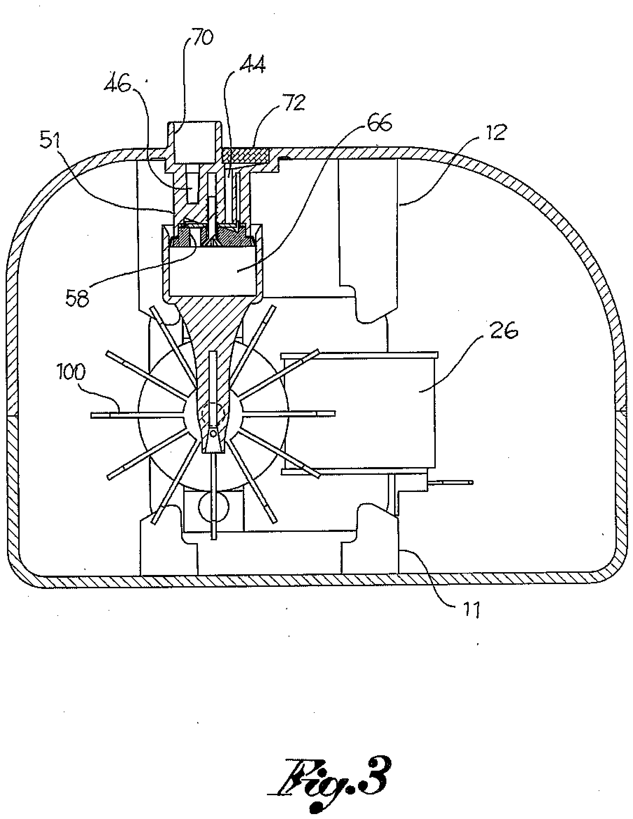

- figure 3 shows the device sectioned at the point of the compression device;

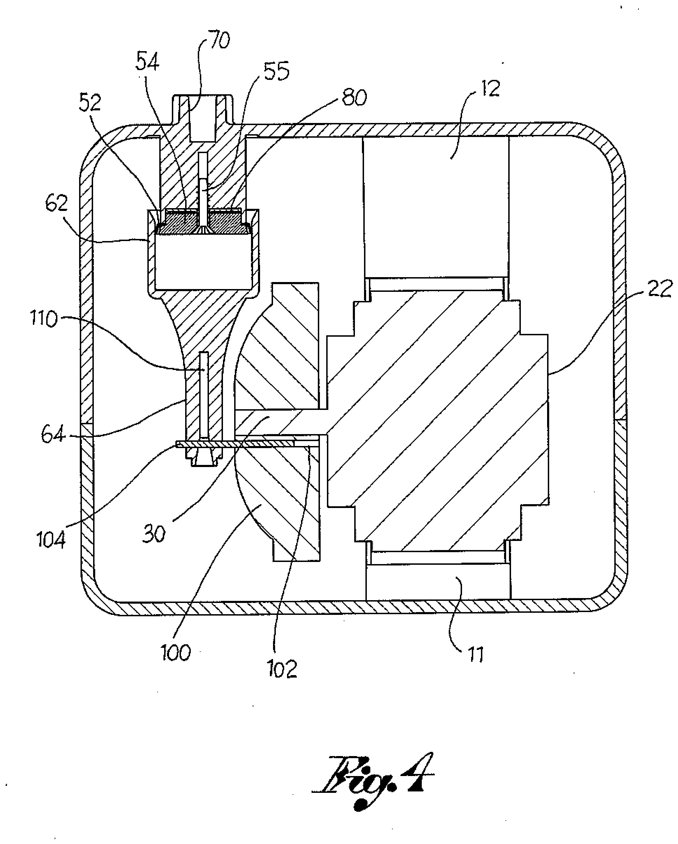

- figure 4 is another cross-section view of the device;

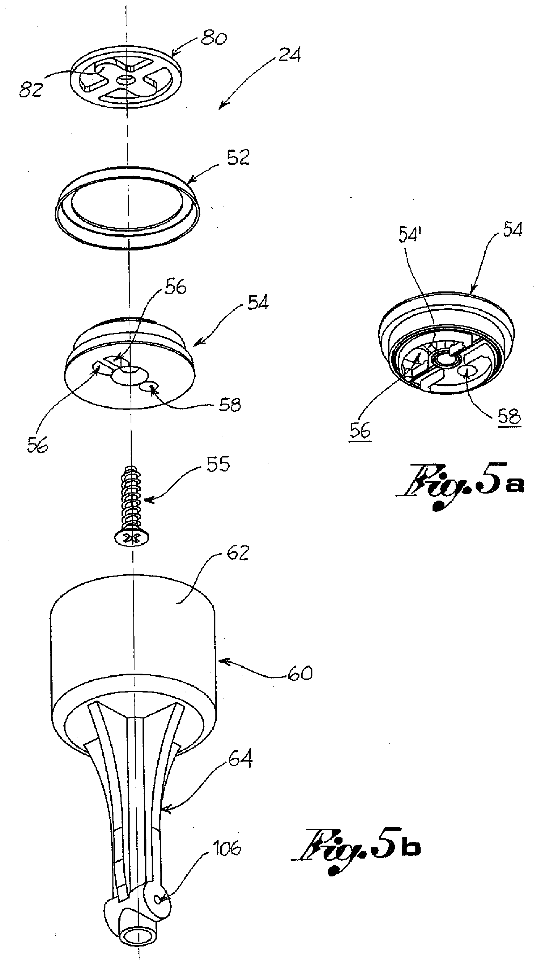

- figure 5a shows an enlarged perspective view of the sealing plug in figure 1;
- figure 5b shows a perspective view of the compressor device, in a perspective opposite to that shown in figure 1; and
- figures 6a and 6b show two corresponding sections of the compressor device in an intake and delivery phase respectively.

In said drawings, reference numeral 1 globally denotes an aerosol-therapy device according to the invention in its entirety.

The device 1 comprises a casing 10, a compressor group 20 mounted in said casing and air intake 40 and delivery 42 means respectively to and from said compressor group 20. The compressor group 20 comprises in turn a motor 22 and a compressor device 24 which can be activated by said motor 22 to draw in air from the outside environment and deliver air to a dispenser device (not shown), respectively through said air intake 40 and delivery means 42.

In one embodiment, the motor 22 is an inductive rotation motor, that is of the type comprising a stator 26 and a rotor 28 which a drive shaft 30 is joined to.

The compressor device 24 comprises a piston 50 fixed to and supported exclusively by the casing 10 and a cylinder 60 sliding on said piston 50 and eccentrically connected to the drive shaft 30 of the motor. Thanks to such connection of the cylinder 60 to the drive shaft 30 by means of an eccentric, the rotation of the drive shaft 30 translates into a substantially rectilinear alternate movement of the cylinder 60 in relation to the piston 50, which is, instead fixed, being joined to the casing 10. In other words, the compressor device 24 is activated by the motor 22 by means of a connecting rod-crank mechanism.

Preferably, the cylinder 60 is connected to a fan 100, described below, eccentrically.

According to a preferred embodiment, the cylinder 60 slides along an annular sealing membrane 52 supported by the piston 50. For example, said annular membrane 52 is made in self-lubricating material.

In a preferred embodiment, the cylinder 60 comprises a hollow head 62, which houses the piston 50 so as to slide, and a stem 64 which extends from said head 62 for connection of the cylinder to the drive shaft 30. The hollow head 62 and the piston 50 define between them a cylinder chamber 66. Advantageously, the stem 64 is made in one piece with the hollow head 62. In other words, head 62 and stem 64 are rigidly connected, without any connection joint. Such design choice considerably simplifies the structure of the compressor device, also in the light of the reduced size of these components, but entails the generation of a slight oscillatory movement of the cylinder in relation to the piston 50. To such purpose, the annular sealing membrane 52 is elastically deformable so as to compensate such oscillatory movement of the cylinder 60. For example, said annular membrane 52 is a "cup" shape, that is it has a lip-shaped peripheral rim, for example facing the cylinder 60.

In a preferred embodiment, the air intake 40 and delivery means 42 comprise intake 44 and delivery 46 passages suitable for placing the cylinder chamber 66 in fluidic communication respectively with the outside environment and with a delivery connector 70 made on the casing 10 and connectable to a delivery pipe (not shown). A check valve 80, hereafter described in greater detail, is joined to each of said intake 44 and delivery passages 46. In addition, a filter 72 is placed along the intake passage 44 at least.

In one embodiment, the piston 50 comprises a piston body 51 which the air intake 44 and delivery passages 46 are made at least partially in and defining a valve seat 53 in which said passages are open towards the cylinder chamber 66. In said seat 53 a valve element 80 is housed which as well as ensuring the fluidic separation of the intake 44 and delivery passages 46, acts as a check valve for each of said passages, as will be explained further below. A sealing plug 54 closes the valve seat 53 so as to hold the valve element 80 in said seat 53. For example, the sealing plug 54 is screwed to the piston body 51 by a single central screw 55. An intake aperture 56 and a delivery aperture 58 are made in the sealing plug 54 able to place the cylinder chamber 66 in fluidic communication respectively and alternatively with the intake 44 and delivery passages 46.

In one advantageous embodiment, the valve element 80 is a membrane comprising two flexible tabs 82, each closing a respective aperture 56, 58 in the sealing plug or a respective passage 44, 46 in the piston body. At either the passage 44, 46 or relative aperture 56, 58, the valve seat 53 or plug 54 has an inclined rib 53', 54' suitable to enable a flexing of the respective tab 82 of the valve element 80, and therefore the opening of the aperture or passage, during the air intake or delivery phase. For example, at the intake passage 44, the valve seat 53 has a flat area, in front of which the plug 54 has the intake aperture 56 crossed by an inclined rib 54'. At the delivery passage 46, the valve seat 53 has an inclined rib 53' which crosses said delivery passage 46. In front of said inclined rib 53', the plug 54 has the delivery aperture 58 open in a flat area of the plug 54, which the other tab 82 normally rests on to close said delivery aperture 58. Centrally, the valve seat 53 and the plug 54 have respective flat areas between which a corresponding central portion of the valve element 80 is held, said flat areas and said central portion being finally crossed by a hole for the passage of the connection screw 55 of the plug 54 to the piston body 51.

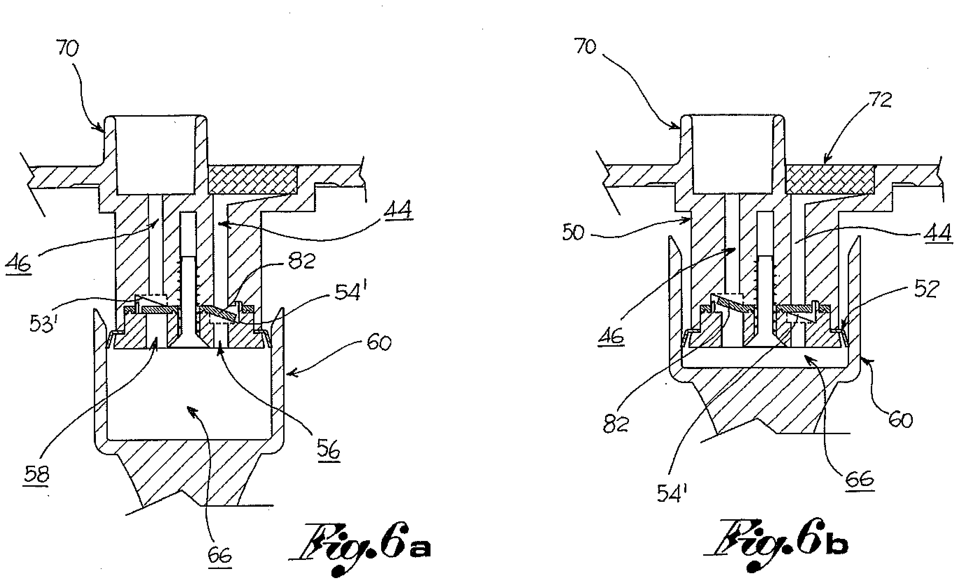

During the intake phase, the tab 82 which is normally placed so as to seal the intake passage 44 flexes as a result of the negative pressure built up in the cylinder until it rests on the rib 54' of the plug, as shown for example in figure 6a. This way the intake passage 44 is placed in fluidic communication with the intake aperture 56 and the air can enter the cylinder chamber 66. In this phase the other tab 82 is kept in contact with the flat area of the plug 54, thereby sealing the delivery aperture 58.

The opposite happens during the delivery phase: the tab 82 sealing the intake passage is pressed harder against the flat portion of the piston body; the other tab 82, normally sealing the delivery aperture 58, is pressed so that it flexes against the piston body 51, until it abuts against the inclined rib 53' of the valve seat 53, as for example shown in figure 6b. At this point, the delivery aperture 58 and the delivery passage 46 are in fluidic communication with each other and the air can be delivered from the cylinder chamber 66 to the dispenser device.

Returning to the annular sealing membrane 52, in one embodiment it is placed and held between the piston body 51 and the sealing plug 54.

In one advantageous embodiment variation, the annular sealing membrane 52 is made in one piece with said plug 54.

According to a preferred embodiment, the device 1 comprises a cooling fan 100 of the compressor group. Said fan is mounted on the drive shaft 30. According to one aspect of the invention, the fan 100 is fitted on the end of the drive shaft 30 projecting from the motor 22 on the side facing the cylinder 60, rather than on the opposite side, as in the compressor groups of the prior art. The fan 100 has a hub 101 where, in an eccentric position in relation to the rotation axis, there is an eccentric hole 102 housing an attachment peg 104 for attachment to the cylinder 60. For example, said peg 104 is inserted in a corresponding hole 106 of the cylinder 60, made in the end of the stem 64.

This position of the fan 100 makes it possible to cool both the motor and the compressor device more efficiently. In addition, the fan itself also acts as an eccentric, thereby making it unnecessary to fit the traditional eccentric with consequent savings in materials and labour costs.

Advantageously, the fan 100 comprises, in a position diametrically opposite the eccentric hole 102, a blade which is thicker than the other blades so as to act as a balancing mechanism for the transmission of the movement from the drive shaft 30 to the cylinder 60.

According to a further aspect of the invention, in the stem 64 of the cylinder 60 there is a lubrication tank 110 open at the end connected to the drive shaft and containing a lubrication liquid which is gradually released in a controlled manner to lubricate the parts in contact between said stem and said drive shaft. For example, said lubrication tank 110 is in the shape of a hole which extends substantially along most of the length of the stem 64 and which opens onto the hole 106 housing the peg 104.

Such tank 110 can be filled easily during the production process and distribute over time the lubricant substance which the transmission mechanism of the movement needs, without the need for specific manual distribution means, such as soaked sponges, stoppers etc.

Going back to the structure of the device 1, according to a preferred embodiment, the piston body 51 is in one piece with the casing 10. The latter may, for example, be made in two pieces, upper 10' and lower 10", the piston body 51 for example being made in the upper part 10', as a cylindrical projection facing inwards to the casing 10.

Advantageously, the two parts 10', 10" of the casing 10 are provided with respective lower 11 and upper 12 projecting seats, for example made in one piece with the casing 10, able to hold the motor 22 firmly. Advantageously, once the motor, fan and cylinder have been assembled such assembly is simply placed on the lower seat 11 of the lower part 10" of the casing 10. After fitting the piston 50 to the valve element 80, the sealing plug 54 and the annular sealing membrane 52, the assembly of the device is completed by simply attaching the upper part 10' of the casing to the lower part 10", taking care to rest the upper seat 12 on the motor 22 and to insert the piston 50 in the hollow head 62 of the cylinder 60. It should be noted that the assembly of the compressor group 20 requires only the screwing of the plug 54 to the piston body 51.

A person skilled in the art may make modifications, adaptations and replacements of elements with others functionally equivalent to the embodiments of the device according to the invention so as to satisfy contingent requirements, while remaining within the sphere of protection of the appended claims. Each of the characteristics described as belonging to a possible embodiment can be realised independently of the other embodiments described.

## Claims

1. Aerosol-therapy device (1), comprising a casing (10), a compressor group (20) mounted in said casing, air intake means (40) and air delivery means (42) respectively to and from said compressor group (20), wherein said compressor group comprises a motor (22) and a compressor device (24) which can be activated by said motor (22) by means of a connecting rod-crank mechanism to draw in and deliver air respectively through said air intake (40) and delivery means (42),
**characterised in that**
- said compressor device comprises a piston (50), which is fixed, being joined to said casing (10), and a cylinder (60) sliding on said piston and eccentrically connected to the drive shaft of the motor,
- the compressor group (20) comprises a cooling fan (100) fitted on the drive shaft (30) of the motor, and
- said cooling fan (100) is fitted on the end of the drive shaft (20) projecting from the motor on the side facing the cylinder (60) and is eccentrically connected to the cylinder (60).

2. Device according to claim 1, wherein the fan has a hub (101) with a hole (102) in an eccentric position in which a peg (104) for attachment to the cylinder (60) is housed.

3. Device according to claim 2, wherein the fan (100) comprises, in a position diametrically opposite the eccentric hole, a blade which is thicker than the other blades so as to act as a balancing mechanism for the transmission of the movement from the drive shaft (30) to the cylinder.

4. Device according to claim 2 or 3, wherein in the stem (64) of the cylinder there is a lubrication tank (110) open at the end connected to the drive shaft and containing a lubrication liquid which is gradually released in a controlled manner to lubricate the parts in contact of said stem and said drive shaft.

5. Device according to any of the claims 2-4, wherein said piston (50) is attached to and supported exclusively by said casing (10).

6. Device according to claim 5, wherein the cylinder slides on an annular sealing membrane (52) supported by the piston (50).

7. Device according to claim 6, wherein the cylinder (60) comprises a hollow head (62) which houses the piston so as to slide and a stem (64) extending from said head for connection of the cylinder to the cooling fan (100).

8. Device according to claim 7, wherein said stem (64) is made in one piece with said hollow head (62), and wherein said annular sealing membrane (52) is elastically deformable so as to compensate the oscillatory movement of the cylinder (60).

9. Device according to any one of the claims 2-8, wherein the air intake (40) and delivery means (42) comprise intake (44) and delivery (46) passages suitable for placing a cylinder chamber (66) in fluidic communication respectively with the outside environment and with a delivery connector (70), connectable to a delivery pipe, to each of said intake and delivery passages being associated a check non-return valve (80).

10. Device according to claim 9, wherein the piston (50) comprises a piston body (51) in which the air intake and delivery passages are made at least partially and defining a valve seat (53) in which said passages are open towards the cylinder chamber, a valve element (80) housed in said seat acting as a check non-return valve for each of said passages and a sealing plug (54) of said valve seat able to hold the valve element in the valve seat and having intake (56) and delivery (58) apertures able to place the cylinder chamber in fluidic communication respectively and alternatively with the intake and delivery passages.

11. Device according to claim 10, wherein the annular sealing membrane (52) is positioned and held between said piston body (51) and said plug (54).

12. Device according to claim 10, wherein the annular sealing membrane (52) is made in one piece with said piston plug (54).

13. Device according to any of the claims 10-12, wherein the valve element (80) is a membrane comprising two flexible tabs (82) each closing a respective aperture (56, 58) in the sealing plug (54) or a respective passage (44, 46) in the piston body (51) and wherein at either the passage or relative aperture the valve seat (53) or plug (54) has an inclined rib (52', 54') suitable to enable flexing of the respective tab and therefore the opening of the aperture or passage, during the air intake or delivery phase.

14. Device according to any of the claims 10-13, wherein the piston body (51) is in one piece with the casing (10).

## Patentansprüche

1. Aerosoltherapievorrichtung (1), umfassend ein Gehäuse (10), eine in dem Gehäuse montierte Kompressorgruppe (20), Lufteinlassmittel (40) und Luftabgabemittel (42) jeweils zu und von der Kompressorgruppe (20), wobei die Kompressorgruppe einen Motor (22) und eine Kompressorvorrichtung (24) umfasst, die von dem Motor (22) aktiviert werden kann, und zwar mittels eines verbindenden Stange-Kurbel-Mechanismus, um Luft durch den Lufteinlass (40) und die Abgabemittel (42) anzusaugen bzw. abzugeben, **dadurch gekennzeichnet, dass**
- die Kompressorvorrichtung einen Kolben (50), der durch Verbundensein mit dem Gehäuse (10) fixiert bzw. befestigt ist, und einen Zylinder (60) umfasst, der an dem Kolben gleitet und exzentrisch mit der Antriebswelle des Motors verbunden ist,
- die Kompressorgruppe (20) ein Kühlgebläse (100) umfasst, das an die Antriebswelle (30) des Motors gepasst ist, und
- das Kühlgebläse (100) an das Ende der Antriebswelle (20) gepasst ist, das von dem Motor auf der dem Zylinder (60) zugewandten Seite vorspringt, und exzentrisch mit dem Zylinder (60) verbunden ist.

2. Vorrichtung nach Anspruch 1, wobei das Gebläse eine Nabe (101) mit einem Loch (102) in einer exzentrischen Position aufweist, in der ein Zapfen (104) zur Anbringung an dem Zylinder (60) untergebracht ist.

3. Vorrichtung nach Anspruch 2, wobei das Gebläse (100) in einer Position diametral gegenüberliegend zu dem Exzenterloch eine Schaufel aufweist, die dicker ist als die anderen Schaufeln, um so als ein Ausgleichsmechanismus für die Übertragung der Bewegung von der Antriebswelle (30) zu dem Zylinder zu wirken.

4. Vorrichtung nach Anspruch 2 oder 3, wobei es in dem Schaft (64) des Zylinders einen Schmiertank (110) gibt, der an dem Ende offen ist, das mit der Antriebswelle verbunden ist, und der eine Schmierflüssigkeit enthält, die allmählich in kontrollierter Weise freigesetzt wird, um die Teile zu schmieren, die in Kontakt mit dem Schaft und der Antriebswelle sind.

5. Vorrichtung nach einem der Ansprüche 2-4, wobei der Kolben (50) an dem Gehäuse (10) angebracht ist und ausschließlich durch dieses gestützt bzw. getragen ist.

6. Vorrichtung nach Anspruch 5, wobei der Zylinder auf einer ringförmigen Dichtungsmembran (52) gleitet, die durch den Kolben (50) gestützt bzw. getragenen ist.

7. Vorrichtung nach Anspruch 6, wobei der Zylinder (60) einen Hohlkopf (62), der den Kolben zum Gleiten unterbringt, und einen Schaft (64) umfasst, der sich von dem Kopf zur Verbindung des Zylinders mit dem Kühlgebläse (100) erstreckt.

8. Vorrichtung nach Anspruch 7, wobei der Schaft (64) einstückig mit dem Hohlkopf (62) hergestellt ist und wobei die ringförmige Dichtungsmembran (52) elastisch verformbar ist, um die oszillierende Bewegung des Zylinders (60) zu kompensieren.

9. Vorrichtung nach einem der Ansprüche 2-8, wobei der Lufteinlass (40) und die Abgabemittel (42) einen Einlass-(44) und einen Abgabe-(46)-Trakt umfassen, die geeignet sind, eine Zylinderkammer (66) jeweils in Fluidverbindung mit der Außenumgebung und mit einem Abgabeverbinder (70) zu versetzen, der mit einem Abgaberohr verbindbar ist, wobei jedem des Einlass- und Abgabetraks ein Rückschlagventil zugeordnet ist.

10. Vorrichtung nach Anspruch 9, wobei der Kolben (50) einen Kolbenkörper (51), in dem der Lufteinlass- und der -abgabetrakt zumindest teilweise ausgebildet sind und einen Ventilsitz (53) definieren, in dem die Trakte zu der Zylinderkammer hin offen sind, ein Ventilelement (80), das in dem Sitz untergebracht ist, der als ein Rückschlagventil für jeden der Trakte wirkt, und einen Dichtungsstopfen (54) des Ventilsitzes umfasst, der in der Lage ist, das Ventilelement in dem Ventilsitz zu halten, und der eine Einlass-(56) und eine Auslass-(58)-Öffnung aufweist, die in der Lage sind, die Zylinderkammer jeweils und alternativ bzw. abwechselnd in Fluidverbindung mit dem Einlass- und dem Auslasstrakt zu versetzen.

11. Vorrichtung nach Anspruch 10, wobei die ringförmige Dichtungsmembran (52) zwischen dem Kolbenkörper (51) und dem Stopfen (54) positioniert und gehalten ist.

12. Vorrichtung nach Anspruch 10, wobei die ringförmige Dichtungsmembran (52) einstückig mit dem Kolbenstopfen (54) hergestellt ist.

13. Vorrichtung nach einem der Ansprüche 10-12, wobei das Ventilelement (80) eine Membran ist, die zwei flexible Laschen (82) umfasst, die jeweils eine entsprechende Öffnung (56, 58) in dem Dichtungsstopfen (54) oder einen entsprechenden Trakt (44, 46) in dem Kolbenkörper (51) verschließen, und wobei entweder an dem Trakt oder der relativen Öffnung der Ventilsitz (53) oder Stopfen (54) eine geneigte Rippe (52', 54') aufweist, die geeignet ist, ein Biegen der jeweiligen Lasche und somit das Öffnen der Öffnung oder des Trakts während der Lufteinlass- oder -abgabephase zu ermöglichen.

14. Vorrichtung nach einem der Ansprüche 10-13, wobei der Kolbenkörper (51) einstückig mit dem Gehäuse (10) ist.

## Revendications

1. Dispositif de thérapie par aérosol (1), comprenant un boîtier (10), un groupe compresseur (20) monté dans ledit boîtier, un moyen d'admission d'air (40) et un moyen de distribution d'air (42) respectivement vers et depuis ledit groupe compresseur (20), dans lequel ledit groupe compresseur comprend un moteur (22) et un dispositif compresseur (24) qui peut être activé par ledit moteur (22) au moyen d'un mécanisme bielle-manivelle de liaison pour aspirer et distribuer l'air respectivement à travers lesdits moyens d'admission (40) et de distribution (42) d'air,
**caractérisé en ce que**
- ledit dispositif compresseur comprend un piston (50), qui est fixé, relié audit boîtier (10), et un cylindre (60) coulissant sur ledit piston et relié excentriquement à l'arbre d'entrainement du moteur,
- le groupe compresseur (20) comprend un ventilateur de refroidissement (100) installé sur l'arbre d'entraînement (30) du moteur, et
- ledit ventilateur de refroidissement (100) est installé sur l'extrémité de l'arbre d'entraînement (20) faisant saillie du moteur sur le côté faisant face au cylindre (60) et est relié excentriquement au cylindre (60).

2. Dispositif selon la revendication 1, dans lequel le ventilateur a un moyeu (101) avec un trou (102) dans une position excentrique dans lequel une cheville (104) à fixer au cylindre (60) est logée.

3. Dispositif selon la revendication 2, dans lequel le ventilateur (100) comprend, dans une position diamétralement opposée au trou excentrique, une pale qui est plus épaisse que les autres pales de sorte à agir comme mécanisme d'équilibrage pour la transmission du mouvement de l'arbre d'entraînement (30) au cylindre.

4. Dispositif selon la revendication 2 ou 3, dans lequel dans la tige (64) du cylindre, il y a un réservoir de lubrification (110) ouvert à l'extrémité reliée à l'arbre d'entraînement et contenant un liquide de lubrification qui est progressivement libéré d'une manière contrôlée pour lubrifier les parties en contact de ladite tige et dudit arbre d'entraînement.

5. Dispositif selon l'une quelconque des revendications 2-4, dans lequel ledit piston (50) est fixé à et supporté exclusivement par ledit boîtier (10).

6. Dispositif selon la revendication 5, dans lequel le cylindre coulisse sur une membrane d'étanchéité annulaire (52) supportée par le piston (50).

7. Dispositif selon la revendication 6, dans lequel le cylindre (60) comprend une tête creuse (62) qui loge le piston de façon à coulisser et une tige (64) s'étendant de ladite tête pour la liaison du cylindre au ventilateur de refroidissement (100).

8. Dispositif selon la revendication 7, dans lequel ladite tige (64) est réalisée d'une seule pièce avec ladite tête creuse (62), et dans lequel ladite membrane d'étanchéité annulaire (52) est élastiquement déformable de façon à compenser le mouvement d'oscillation du cylindre (60).

9. Dispositif selon l'une quelconque des revendications 2-8, dans lequel les moyens d'admission (40) et de distribution (42) d'air comprennent des passages d'admission (44) et de distribution (46) adaptés pour placer une chambre de cylindre (66) en communication fluidique respectivement avec l'environnement extérieur et avec un connecteur de distribution (70), pouvant être relié à un tuyau de distribution, un clapet anti-retour (80) étant associé à chacun desdits passages d'admission et de distribution.

10. Dispositif selon la revendication 9, dans lequel le piston (50) comprend un corps de piston (51) dans lequel les passages d'admission et de distribution d'air sont réalisés au moins partiellement et définissant un siège de soupape (53) dans lequel lesdits passages sont ouverts vers la chambre cylindrique, un élément de soupape (80) logé dans ledit siège agissant en tant que clapet anti-retour pour chacun desdits passages et un bouchon d'étanchéité (54) dudit siège de soupape capable de maintenir l'élément de soupape dans le siège de soupape et ayant des ouvertures d'admission (56) et de distribution (58) capables de placer la chambre de cylindre en communication fluidique respectivement et alternativement avec les passages d'admission et de distribution.

11. Dispositif selon la revendication 10, dans lequel la membrane d'étanchéité annulaire (52) est positionnée et maintenue entre ledit corps de piston (51) et ledit bouchon (54).

12. Dispositif selon la revendication 10, dans lequel la membrane d'étanchéité annulaire (52) est réalisée d'une seule pièce avec ledit bouchon de piston (54).

13. Dispositif selon l'une quelconque des revendications 10-12, dans lequel l'élément de soupape (80) est une membrane comprenant deux pattes souples (82) fermant chacune une ouverture respective (56, 58) dans le bouchon d'étanchéité (54) ou un passage respectif (44, 46) dans le corps de piston (51) et dans lequel au niveau soit du passage soit de l'ouverture relative, le siège de soupape (53) ou bouchon (54) a une nervure inclinée (52', 54') adaptée pour permettre la flexion de la patte respective et donc l'ouverture de l'ouverture ou du passage, pendant la phase d'amission ou de distribution d'air.

14. Dispositif selon l'une quelconque des revendications 10-13, dans lequel le corps de piston (51) est d'une seule pièce avec le boîtier (10).
